# FASCICULE DE BREVET EUROPEEN

(11) **EP 1 226 148 B1**
(45) Date de publication et mention de la délivrance du brevet: **23.11.2011**
(21) Numéro de dépôt: 00969634.5
(22) Date de dépôt: 18.10.2000
(51) Int. Cl.: C07H 19/01, C08B 37/00, A61K 31/70, A61P 25/02, A61P 25/14, A61P 25/16, A61P 25/28, A61P 19/02, A61P 9/10, A61P 31/18

(54) **NOUVEAUX OLIGOSACCHARIDES, LEUR PREPARATION ET LES COMPOSITIONS PHARMACEUTIQUES LES CONTENANT**
NEUE OLIGOSACCHARIDE, VERFAHREN ZU DEREN HERSTELLUNG UND DIESE ENTHALTENDE PHARMAZEUTISCHE ZUSAMMENSETZUNGEN
NOVEL OLIGOSACCHARIDES, PREPARATION METHOD AND PHARMACEUTICAL COMPOSITIONS CONTAINING SAME

(30) Priorité: 22.10.1999 FR 9913182
(43) Date de publication de la demande: 31.07.2002
(73) Titulaire: Aventis Pharma S.A., 92160 Antony (FR)
(72) Inventeur: MOURIER, Pierre, F-94220 Charenton le Pont (FR); PERRIN, Elisabeth, F-27000 Evreux (FR); VISKOV, Christian, F-91130 Ris Orangis (FR); STUTZMANN, Jean-Marie, F-94440 Villecresnes (FR); WAHL, Florence Chez M. Karoby, F-75004 Paris (FR)
(74) Mandataire: Lecocq, Fabrice
(86) Numéro de dépôt international: PCT/FR2000/002897
(87) Numéro de publication internationale: WO 2001/029055

(56) Documents cités:
- EP-A- 0 084 999
- ICHIKAWA Y. ET AL.: "Synthesis, from cellobiose, of a trisaccharide closely related to the GlcNAc->GlcA->GlcN segment of the anti-thrombin-binding sequence of heparin" CARBOHYDRATE RESEARCH, vol. 141, 1985, pages 273-282, XP002142682 cité dans la demande
- WESSEL H. P.: "Sulfated 1,6-anhydro-4-O-(beta-D-glucopyranosyluron ate)-beta-D-glucopyranosyl derivatives: syntheses and conformations" J. CARBOHYDRATE CHEMISTRY, vol. 11, no. 8, 1992, pages 1039-1052, XP000929166 cité dans la demande

## Description

La présente invention concerne des oligosaccharides de formule : 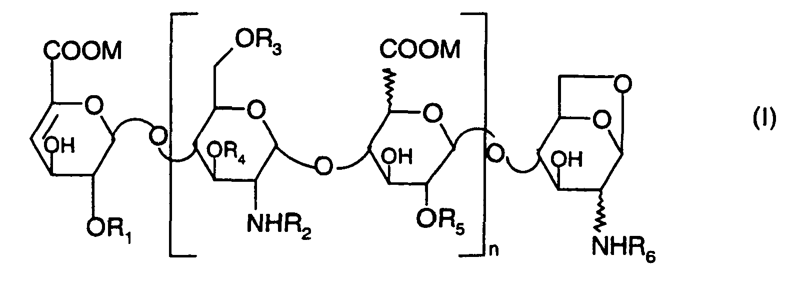 leurs mélanges, leurs diastéréoisomères, leur procédé de préparation et les compositions pharmaceutiques les contenant.

Des disaccharides sulfatés possédant en extrémité réductrice une structure 1,6-anhydro ont été décrits par H.P. WESSEL, J. Carbohydrate Chemistry, 11(8), 1039-1052 (1992); aucune activité pharmacologique n'est mentionnée pour ceux-ci.

Des trisaccharides sulfatés comportant un motif 1,6-anhydro ont également été décrits dans le brevet EP84999 et par Y. ICHIKAWA et coll., Carbohydr. Res, 141, 273-282 (1985) comme intermédiaires pour la préparation d'oligosaccharides supérieurs. Ces trisaccharides ont une faible activité anti-Xa.

Dans la formule (I) n est un entier de 0 à 25, R₁, R₃, R₄ et R₅, identiques ou différents, représentent un atome d'hydrogène ou un radical SO₃M, R₂ et R₆, identiques ou différents, représentent un atome d'hydrogène ou un radical SO₃M ou COCH₃ et M est sodium, calcium, magnésium ou potassium.

Ces oligosaccharides comportent ainsi un nombre pair de saccharides.

Dans la formule (I), R₄ est, de préférence, un atome d'hydrogène.

De façon préférentielle, n est un entier de 0 à 10 et, en particulier de 0 à 6 et encore plus particulièrement de 1 à 6.

Les oligosaccharides de formule (I) peuvent être préparés par action d'un hydroxyde de métal alcalin ou d'ammonium quaternaire sur des oligosaccharides de formule : 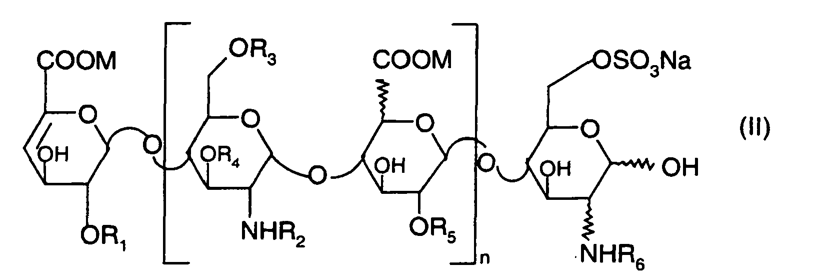 dans laquelle n est un entier de 0 à 25, R₁, R₃, R₄ et R₅, identiques ou différents, représentent un atome d'hydrogène ou un radical SO₃M, R₂ et R₆, identiques ou différents, représentent un atome d'hydrogène ou un radical SO₃M ou COCH₃ et M est sodium, calcium, magnésium ou potassium, ou un mélange de ceux-ci.

Cette réaction s'effectue en milieu aqueux, à une température de 40 à 80°C, à un pH de 10 à 13.

Comme hydroxyde de métal alcalin qui peut être utilisé, on peut citer la soude, la potasse, l'hydroxyde de lithium et l'hydroxyde de césium.

Comme hydroxyde d'ammonium quaternaire qui peut être utilisé, on peut citer l'hydroxyde de tétrabutylammonium.

La quantité d'hydroxyde de métal alcalin ou d'ammonium quaternaire doit être suffisante pour que le pH du milieu réactionnel reste stable durant toute la durée de la réaction. Il est ainsi nécessaire d'ajouter en continu tout au long de la réaction l'hydroxyde de métal alcalin ou d'ammonium quaternaire.

De préférence, l'hydroxyde de métal alcalin ou d'ammonium quaternaire est sous forme de solution aqueuse de 1 à 5%.

De façon préférentielle, la réaction s'effectue à une température de 60 à 70°C.

Avantageusement, le pH réactionnel est de 11 à 12,5.

La réaction est arrêtée par acidification du milieu réactionnel, par exemple par addition de résine acide telle que la résine Amberlite IR120^{R} (Fluka).

Les oligosaccharides de formule (I) peuvent être éventuellement purifiés par chromatographie par perméation de gel de type polyacrylamide-agarose tel que celui commercialisé sous la marque Ultrogel ACA202^{R} (Biosepra) selon le protocole décrit ci-dessous pour la séparation des oligosaccharides intermédiaires de formule (II). Les oligosaccharides de formule (I) pour lesquels n est 0 ou 1 peuvent aussi éventuellement être purifiés sur colonne d'alumine avec comme éluant un mélange eau-éthanol.

Les oligosaccharides intermédiaires de formule (II) et leurs mélanges peuvent être obtenus par séparation par chromatographie sur gel d'un mélange d'oligosaccharides (III) obtenu par dépolymérisation enzymatique de l'héparine ou dépolymérisation basique de l'ester benzylique de l'héparine ou d'un ester benzylique d'héparine d'hémisynthèse.

Cette chromatographie s'effectue sur des colonnes remplies de gel de type polyacrylamide-agarose tel que celui commercialisé sous la marque Ultrogel ACA202^{R} (Biosepra). De préférence, on utilise une batterie de colonnes de gel polyacrylamide agarose. Le nombre de colonnes utilisées est adapté en fonction du volume, du gel et des oligosaccharides à séparer. Le mélange est élué par une solution contenant un tampon phosphate et du chlorure de sodium. De préférence, la solution tampon phosphate est une solution à 0,02 mol/l de NaH₂PO₄/Na₂HPO₄ (pH 7) contenant 0,1 mol/l de chlorure de sodium. La détection des différentes fractions est réalisée par spectrométrie UV (254 nm) et ionique (IBF). Les fractions ainsi obtenues peuvent ensuite être éventuellement purifiées par exemple par chromatographie SAX (strong anion exchange) selon les méthodes connues de l'homme du métier et notamment selon les méthodes décrites par K.G. Rice et R.J Linhardt, Carbohydrate Research 190, 219-233 (1989), A. Larnkjaer, S.H. Hansen et P.B. Ostergaard, Carbohydrate Research, 266, 37-52 (1995) et dans le brevet WO90/01501 (exemple 2). Les fractions sont ensuite lyophilisées puis désalées sur une colonne remplie de gel telle qu'une colonne de gel Séphadex G10^{R} ( Pharmacia Biochemicals).

Lorsque la purification n'est pas réalisée par chromatographie SAX, les lyophilisats peuvent être éventuellement purifiés par précipitation simple ou fractionnée selon les méthodes connues de l'homme du métier et notamment selon la méthode décrite dans le brevet FR2548672. De façon générale, on opère selon le protocole suivant :

La fraction lyophilisée à purifier est dissoute à 25°C dans environ dix volumes d'eau distillée. Par ajout de méthanol ou d'éthanol, on fait précipiter l'oligosaccharide désiré en contrôlant son enrichissement par chromatographie CLHP (Chromatographie Liquide Haute Performance). L'ajout de méthanol ou d'éthanol est déterminé en fonction de la pureté et du rendement désiré du dit oligosaccharide. De même, cette opération peut être réalisée en plusieurs étapes successives à partir de la solution initiale de lyophilisat. Pour celà, on rajoute l'agent insolubilisant (méthanol ou éthanol) par petites portions et on isole après chaque ajout le précipité obtenu. Les précipités ainsi préparés sont analysés par chromatographie CLHP. Selon la pureté et le rendement désiré, on rassemble les fractions adéquates de précipité.

Pour les intermédiaires de formule (II) pour lesquels n = 0, 1 ou 2, il est préférable de partir d'un mélange (III) obtenu par dépolymérisation enzymatique.

Cette dépolymérisation s'effectue au moyen d'héparinase I (EC 4.2.2.7), au sein d'une solution tampon phosphate de pH7, en présence de chlorure de sodium et de BSA (Albumine de Sérum Bovin), à une température comprise entre 10 et 18°C et, de préférence 15°C, pendant 8 à 10 jours et, de préférence, 9 jours. La dépolymérisation est arrêtée par exemple par chauffage du milieu réactionnel à 100°C pendant 2 minutes et le mélange est récupéré par lyophilisation. Il est préférable d'utiliser 7UI d'héparinase I pour 25 g d'héparine. La solution tampon phosphate comprend généralement 0,05 mol/l de NaH₂PO₄/Na₂HPO₄ (pH 7) en présence de 0,1 mol/l de chlorure de sodium. La concentration en BSA est généralement de 2%.

Pour les intermédiaires de formule (II) pour lesquels n = 0, 1, 2, 3 ou 4, il est préférable de partir d'un mélange (III) obtenu par dépolymérisation d'un ester benzylique de l'héparine.

L'ester benzylique de l'héparine peut être préparé selon les méthodes décrites dans les brevets US5389618, EP40144, FR2548672. Le taux d'estérification sera de préférence compris entre 50 et 100 %. De façon préférentielle, il sera compris entre 70 et 90%.

La dépolymérisation s'effectue en milieu aqueux, au moyen d'un hydroxyde de métal alcalin (hydroxyde de lithium, soude, potasse ou hydroxyde de césium par exemple) ou d'un hydroxyde d'ammonium quaternaire (hydroxyde de tétrabutylammonium par exemple), de préférence, à une molarité comprise entre 0,1 et 0,2 mol/l, à une température comprise entre 40 et 80°C, pendant 5 à 120 minutes. Dans un mode préféré, on opère pendant 5 à 15 minutes, à une température comprise entre 60 et 70°C, avec une solution d'hydroxyde de sodium 0,15 mol/l. La réaction de dépolymérisation est arrêtée par neutralisation par addition d'un acide tel que l'acide acétique. Après addition de 10% en poids par volume d'acétate de sodium, le mélange d'oligosaccharides est précipité par addition de méthanol, de préférence, 2 volumes pour 1 volume de milieu réactionnel et filtré.

Selon un aspect préféré de l'invention, le mélange d'oligosaccharides obtenu après dépolymérisation chimique, sous forme d'une solution aqueuse, est enrichi par ultrafiltration sur membranes avec un seuil de coupure nominal approprié (type Pellicon en cellulose régénérée commercialisées par Millipore); le type de membrane étant adapté en fonction du type d'oligosaccharides enrichis à récupérer. Pour les oligosaccharides (II) pour lesquels n = 0, on utilisera une membrane de seuil de coupure nominal de 1 kDa, pour les oligosaccharides (II) pour lesquels n = 1, on utilisera une membrane 1 kDa ou 3 kDa, pour les oligosaccharides (II) pour lesquels n = 2, on utilisera une membrane 3 kDa, pour les oligosaccharides (II) pour lesquels n = 3 ou 4, on utilisera une membrane 5 kDa. Au cours de cette opération, le perméat est récupéré et le rétentat rejeté. Ainsi, la fraction de produit enrichi peut représenter de 50 à 10% du mélange d'oligosaccharides initial tout en conservant au moins 80% de l'oligosaccharide désiré.

Pour les intermédiaires de formule (II) pour lesquels n = 0 à 25, il est préférable de partir d'un mélange (III) obtenu par dépolymérisation d'un ester benzylique de polysaccharide sulfaté d'hémisynthèse. L'ester benzylique de polysaccharide sulfaté d'hémisynthèse est préparé à partir de polysaccharides sulfatés d'hémisynthèse obtenus à partir du polysaccharide K5 et selon les méthodes décrites dans les brevets WO94/29352 et WO96/14425. Les conditions d'estérification, de dépolymérisation et de récupération sont les mêmes que celles décrites précédemment pour l'ester benzylique d'héparine.

Dans tous les procédés précédents, l'héparine initiale peut être d'origine porcine, ovine, caprine ou bovine et peut provenir des mucus, poumons ou peaux des animaux. De préférence, on utilise une héparine de mucus porcin, ovin ou de poumons de boeuf et encore plus préférentiellement de mucus porcin.

Les oligosaccharides de formule (I) présentent des propriétés anti-inflammatoires et peuvent ainsi être utilisées pour la prévention ou le traitement de maladies liées à un processus inflammatoire impliquant la production de substances cytotoxiques telle que le monoxyde d'azote (NO) dont la forme inductible est libérée notamment par les neutrophiles ou les macrophages lorsque ceux-ci migrent et sont activés au niveau d'un tissu. L'activation, la migration, l'adhésion et l'infiltration des neutrophiles se produit au niveau des zones tissulaires ischémiées à la suite d'une occlusion ou d'un spasme d'une artère vascularisant ce tissu. Ces ischémies peuvent se produire soit au niveau cérébral (accident vasculaire cérébral), soit au niveau du myocarde (infarctus du myocarde), soit au niveau des membres inférieurs (ischémies dites périphériques). Les oligosaccharides de formule (I) peuvent ainsi être utilisées pour la prévention et/ou le traitement des maladies neurodégénératives pour lesquelles l'inflammation cérébrale joue un rôle délétère pouvant conduire à la mort parmi lesquelles on peut citer les ischémies cérébrales, les ischémies cardiaques (infarctus du myocarde), les ischémies périphériques, les traumatismes du système nerveux central et notamment les traumatismes crâniens, spinaux et cranio-spinaux, la sclérose en plaques, les douleurs neuropathiques et les neuropathies périphériques, les maladies du motoneurone dont la sclérose latérale amyotrophique, le neuro-sida, la maladie d'Alzheimer, la maladie de Parkinson et la Chorée de Huntington et certaines formes d'ostéoarthrites notamment à localisation articulaire.

L'activité anti-inflammatoire de ces produits est démontrée in vivo dans le test de production de NOx (nitrite et nitrate) induite par un lipopolysaccharide (LPS) provenant d'E. Coli selon le protocole décrit par M. YAMASHITA et coll., Eur. J. Pharmacol, 338, 2, 151-158 (1997) ou J.E. SHELLITO et coll. Am. J. Respir. Cell Mol. Biol., 13, 1, 45-53 (1995).

On injecte, à des souris CD1 mâles (Charles River, 25-35g), à T0 par voie intraveineuse bolus 0,5 mg/kg de l'oligosaccharide, à T+15 minutes, par voie sous-cutanée 1 ou 2 mg/kg de l'oligosaccharide. A T+30 minutes, on administre 100 mg/kg de lipopolysaccharide (LPS) provenant d'E. Coli (Sigma L3129, sérotype 0127:B8). A T+3 heures on injecte à nouveau par voie sous-cutanée 1 ou 2 mg/kg de l'oligosaccharide. A T+5 heures 30 minutes, un échantillon de sang est récupéré par ponction à l'oeil et les concentrations en NOx (nitrite et nitrate) dans le plasma sont déterminées avec la méthode colorimétrique de Griess après réduction du nitrate en nitrite par nitrate réductase de la manière suivante : 12 µl de l'échantillon de plasma sont mélangés avec 88 µl d'eau déionisée et incubés dans le noir 1 heure à température ambiante avec 40 µl de tampon phosphate (0,31 M, pH 7,5), 20 µl de β-NADPH (nicotinamide adénine dinucléotide phosphate réduit) (0,86mM), 20 µl de FDA (flavine adénine dinucléotide) (0,11 mM) et 20 µl de nitrate réductase (2U/ml) (Boehringer Mannheim). 10 µl de ZnSO₄ (1M) sont ajoutés pour précipiter les proteines et après mélange, les échantillons sont centrifugés à 20 000g pendant 5 minutes. Finalement, 50 µl du supernageant sont incubés 10 minutes à température ambiante avec 100 µl du réactif de Griess (sulfanilamide à 1 % dans un mélange acide phosphorique/naphtyéthylènediamine 0,1% dans l'eau déionisée (V/V)). Les densités optiques sont lues à 540 nm avec un spectrophotomètre microplaques; chaque point étant déterminé 2 fois. KNO₃ et NaNO₂ sont utilisés comme standard pour la méthode colorimétrique.

Dans ce test, les oligosaccharides selon l'invention inhibent à plus de 50 % la formation de NOx.

Parmi les oligosaccharides de formule (I) préférés, on peut notamment citer les oligosaccharides pour lesquels :
- n est égal à 0, R₁ et R₆ représentent un radical SO₃Na et M est sodium et le mélange de ses diastéréoisomères
- n est égal à 1, R₁, R₂, R₃, R₅, R₆, représentent un radical SO₃Na, R₄ représente un atome d'hydrogène et M est sodium et le mélange de ses diastéréoisomères
- n est égal à 2, R₁, R₂, R₃, R₅, R₆ représentent un radical SO₃Na, R₄ représente un atome d'hydrogène et M est sodium et le mélange de ses diastéréoisomères.
- n est égal à 2, R₁, R₂, R₃, R₆, représentent un radical SO₃ Na, R₅ représente un atome d'hydrogène ou un radical SO3Na, R₄ représente un atome d'hydrogène et M est sodium et le mélange de ses diastéréoisomères (dérivé 1,6 anhydro ΔIs-Is-IIs).

Les exemples suivants sont représentatifs de la préparation des oligosaccharides de formule (I) et des intermédiaires.

Dans ces exemples les significations des abréviations sont les suivantes :
ΔIs : (acide 4-déoxy-2-*O*-sulfo-α-L-thréo-hex-enopyranosyluronique)-(1→4)-2-déoxy-2-sulfoamino-6-*O*-sulfo-α-D-glucopyranose, sel de tétrasodium ou bien ΔUAp2S-(1→4)-α-D-GlcNp2S6S
Is : (acide 2-sulfo-α-L-idopyranosyluronique)-(1→4)-2-déoxy-2-sulfoamino-6-*O-*sulfo-α-D-glucopyranose, sel de tétrasodium ou bien α-L-IdoAp2S- (1→4)-α-D-GlcN*p*2S6S
IIs : (acide α-L-idopyranosyluronique)-(1→4)-2-déoxy-2-sulfoamino-6-*O*-sulfo-α-D-glucopyranose, sel de trisodium ou bien α-L-IdoA*p*- (1→4)-α-D-GlcN*p*2S6S
IIIs : (acide 2-sulfo-α-L-idopyranosyluronique)-(1→4)-2-déoxy-2-sulfoamino-α-D-glucopyranose, sel de trisodium ou bien α-L-IdoA*p*2S- (1→4)-α-D-GlcN*p*2S
IdoAp : acide idopyranosyluronique
GlcNp : 2-déoxy-2-aminoglucopyranose
ΔUa*p* : acide 4-déoxy-α-L-thréo-hex-enopyranosyluronique
S : sulfate

### EXEMPLES DE PREPARATION DES MELANGES DE FORMULE (II)

### EXEMPLE A - préparation des oligosaccharides de formule (II) pour lesquels n=0, 1 et 2 par dépolymérisation enzymatique et séparation

Dissoudre 25 g d'héparine dans 250 ml d'une solution tampon phosphate contenant 0,05 mol/l NaH₂PO₄/Na₂HPO₄ (pH = 7), 0,2 mol/l de chlorure de sodium et 2 % de BSA (Albumine de Sérum Bovin). On introduit dans le mélange 7 UI d'héparinase I (EC 4.2.2.2.7) et la solution obtenue est refroidie à 15°C puis maintenue à cette température pendant toute la durée de la réaction de dépolymérisation. L'avancement de la réaction est suivie par des prélèvements échelonnés d'aliquots et analysés par chromatographie sur perméation de gel. Au bout de 9 jours, la réaction enzymatique est arrêtée en chauffant le milieu réactionnel à 100°C pendant deux minutes. Le mélange refroidi est ensuite lyophylisé. On obtient ainsi un mélange d'oligosaccharides (III).

Le mélange d'oligosaccharides (III) obtenu est ensuite chromatographié selon la méthode suivante : La chromatographie s'effectue sur des colonnes remplies avec du gel polyacrylamide-agarose connu sous la dénomination Ultrogel ACA 202 et le mélange est élué par une solution contenant un tampon phosphate (0,02 mol/l NaH₂PO₄/Na₂HPO₄) pH = 7 et 0,1 mol/l de chlorure de sodium. La détection est réalisée en spectrométrie UV (254 nm) et ionique (IBF). Les produits peuvent être éventuellement purifiés par chromatographie SAX (strong anion exchange) ou par précipitation fractionnée selon la méthode décrite dans le brevet FR 2548672. Les fractions de produit récupéré sont lyophilisées puis désalées sur une colonne remplie de gel sephadex G10^{R} (Pharmacia Biochemicals).

Par cette méthode, on obtient 3 g de disaccharide Is, 1100 mg d'un mélange d'hexasaccharide contenant typiquement 55 % de dérivé ΔIs-Is-Is , 35 % de ΔIs-Is-IIs et 10 % de ΔIs-Is-IIIs. Ce dernier mélange peut être purifié selon les méthodes connues de l'homme du métier pour en séparer chacun des constituants ou utilisé tel quel pour la transformation en dérivés 1,6 anhydro de formule (I). Il est à noter qu'au cours de cette transformation l'hexasaccharide ΔIs-Is-IIIs ne peut conduire à la formation de composés de formule (I).

### EXEMPLE B - préparation des oligosaccharides de formule (II) pour lesquels n=0, 1, 2, 3 ou 4 par dépolymérisation de l'ester benzylique d'héparine et séparation

### a - Préparation de l'ester benzylique de l'héparine

L'ester benzylique de l'héparine est préparé selon l'exemple 3 du brevet US 5,389,618.

### b- Dépolymérisation

Dissoudre 100 g d'ester benzylique de l'héparine dans 1,9 1 d'eau déminéralisée. Le mélange est porté à 60°C sous agitation. Après l'obtention d'une solution homogène, on introduit en une seule fois environ 35 ml d'une solution d'hydroxyde de sodium à 23 %. Après 10 minutes de réaction, la solution est ensuite refroidie et neutralisée par 80 ml d'une solution d'acide acétique environ 2 N. A cette solution, on ajoute 10 % en poids/volume d'acétate de sodium. Le mélange d'oligosaccharides est précipité par addition d'environ 2 volumes de méthanol. Le précipité est isolé par filtration, lavé au méthanol à deux reprises puis séché sous pression réduite à 50°C. Après séchage, on obtient 73,8 g d'un mélange d'oligosaccharides (II).

### c- Enrichissement en oligosaccharide pour lequel n = 1

Dissoudre 30 g du mélange d'oligosaccharides obtenu précédemment dans environ 35 volumes d'eau. Cette solution est ultrafiltrée sur membrane 3 kDa (Pellicon). Lorsque 600 ml de perméat on été soutirés, on dilue le rétentat par 500 ml d'eau. L'opération est poursuivie jusqu'à soutirement de 450 ml de perméat supplémentaires. Les deux fractions de perméat sont réunies puis concentrées à sec sous pression réduite. On obtient 6,1 g d'un solide blanc jaunâtre. L'analyse du solide par chromatographie de perméation sur gel indique qu'il contient environ 30 % d'oligosaccharide de formule (II) pour lequel n=1.

### d - Fractionnement des mélanges d'oligosaccharides ultrafiltrés

Le mélange enrichi est fractionné sur des colonnes remplies avec du gel polyacrylamide-agarose connu sous la dénomination Ultrogel ACA 202 (on utilise 4 colonnes en série d'un diamètre de 10 cm et d'une hauteur de 50 cm). 5 g du mélange enrichi par ultrafiltration sont dissous dans 25 ml d'eau puis élués par une solution 0,2 mol/l de chlorure de sodium à la vitesse de 5 ml/min. On recueille en bas de colonne des fractions de 25 ml. La détection des produits est réalisée en spectrométrie UV (254 nm) et ionique (IBF). Les fractions de produit pour lequel n = 1 sont récupérées, lyophilisées puis désalées sur une colonne remplie de gel Sephadex G 10. Après lyophilisation, on obtient 1 g de tétrasaccharide contenant typiquement 70 % de dérivé ΔIs-Is de formule II (R₁, R₂, R₃, R₅, R₆ = SO₃Na; R₄ = H et M = Na). Le dérivé ΔIs-Is peut être éventuellement purifié par chromatographie SAX (strong anion exchange) ou selon un aspect preférentiel par précipitation fractionnée selon la méthode décrite dans le brevet FR 2548672.

### EXEMPLE 1

Dans un réacteur maintenu à 66°C, on introduit 5 ml d'une solution d'hydroxyde de sodium à 0,0063 mol/l. Le pH de la solution est alors mesuré et pris comme valeur cible (pH = 11,35). Sous agitation, on ajoute en une fois 30 mg de l'oligosaccharide de formule (II) pour lequel n est égal à 0, R₁ et R₆ représentent un radical SO₃Na et M est sodium. Le pH est alors régulé et maintenu à pH 11,35 par addition continue d'une solution d'hydroxyde de sodium à 0,5 mol/l. Après 10 heures, on arrête l'ajout d'hydroxyde de sodium et le mélange réactionnel est refroidi à 25°C. Le pH de la solution est alors ramené entre 6 et 7 par addition de résine Amberlite IR120. Le mélange est filtré sur membrane Whatman GF/B puis concentré à sec sous pression réduite (2,7 kPa), à une température voisine de 25°C. Le produit est repris avec 0,5 ml d'eau distillée puis lyophilisé. On obtient ainsi 29 mg d'un mélange de diastéréoisomères de l'oligosaccharide de formule (I) pour lequel n est égal à 0, R₁ et R₆ représentent un radical SO₃Na et M est sodium [(acide 4-déoxy-2-*O*-sulfo-α-L-thréo-hex-4-enopyranosyluronique-(1→4)-1,6-anhydro-2-déoxy-2-sulfoamino-β-D-mannopyranose, sel de trisodium) : Spectre proton dans D₂O, 400MHz, T=298K, δ en ppm: 3,15 (1H, s, H2), 3,75 (2H, m, H6 et H3), 3,88 (1H, m, H4), 4,20 (1H, d, J=8Hz, H6), 4,22 (1H, t, J=5Hz, H3'), 4,58 (1H, m, H2'), 4,75 (1H, m, H5), 5,53 (1H, s, H1), 5,60 (1H, dd, J=6 et 1Hz, H l'), 6,03 (1H, d, J=5Hz, H4'); (acide 4-déoxy-2-*O*-sulfo-α-L-thréo-hex-4-enopyranosyluronique-(1→4)-1,6-anhydro-2-déoxy-2-sulfoamino-β-D-glucopyranose, sel de trisodium) : Spectre proton dans D₂O, 400MHz, T=298K, δ en ppm: 3,34 (1H, dd, J=7 et 2Hz, H2), 3,72 (1H, t, J=8Hz, H6), 3,90 (1H, m, H3), 4,03 (1H, s, H4), 4,20 (1H, d, J=8Hz, H6), 4,23 (1H, t, J=5Hz, H3'), 4,58 (1H, m, H2'), 4,78 (1H, m, H5), 5,50 (1H, s, H1), 5,60 (1H, dd, J=6 et 1Hz, H1'), 6,03 (1H, d, J=5Hz, H4')].

### EXEMPLE 2

Dans un réacteur maintenu à 62°C, on introduit 33,3 ml d'une solution d'hydroxyde de sodium à 0,0063 mol/l. Le pH de la solution est alors mesuré et pris comme valeur cible (pH = 11,15). Sous agitation, on ajoute en une fois 200 mg de l'oligosaccharide de formule (II) pour lequel n est égal à 1, R₁, R₂, R₃, R₅ et R₆ représentent un radical SO₃Na, R₄ représente un atome d'hydrogène et M est sodium. Le pH est alors régulé et maintenu à pH 11,15 par addition continue d'une solution d'hydroxyde de sodium à 0,5 mol/l. Après 12 heures, on arrête l'ajout d'hydroxyde de sodium et le mélange réactionnel est refroidi à 25°C. Le pH de la solution est alors ramené entre 6 et 7 par addition de résine Amberlite IR120. Le mélange est filtré sur membrane Whatman GF/B puis concentré à sec sous pression réduite (2,7 kPa), à une température voisine de 25°C. Le produit est repris avec 3 ml d'eau distillée puis lyophilisé. On obtient ainsi 230 mg de l'oligosaccharide de formule (I) pour lequel n est égal à 1, R₁, R₂, R₃, R₅, R₆, représentent un radical SO₃Na, R₄ représente un atome d'hydrogène et M est sodium, sous forme d'un mélange des diastéréoisomères [(acide 4-déoxy-2-*O*-sulfo-α-L-thréo-hex-4-enopyranosyluronique-(1→4)-2-déoxy-2-sulfoamino-6-*O*-sulfo-α-D-glucopyranosyl-(1→4)-acide 2-*O*-sulfo-α-L-idopyranosyluronique -(1→4)-1,6-anhydro-2-déoxy-2-sulfoamino-β-D-mannopyranose, sel d'heptasodium): Spectre proton dans D₂O, 400MHz, T=298K, δ en ppm: 3,15 (1H, s, H2), 3,25 (1H, m, H2"), 3,60 (1H, m, H3"), entre 3,70 et 4,70 (14H, massif, H3/H4/H6, H2'/H3'/H4'/H5', H4"/H5"/H6", H2"'/H3"'), 4,75 (1H, m, H5), entre 5,20 et 5,40 (2H, m, H1' et H1"), 5,45 (1H, m, H1"'), 5,56 (1H, m, H1), 5,94 (1H, d, J=5Hz, H4); (acide 4-déoxy-2-*O-*sulfo-α-L-thréo-hex-4-enopyranosyluronique -(1→4)-2-déoxy-2-sulfoamino-6-*O-*sulfo-α-D-glucopyranosyl-(1→4) acide -2-*O*-sulfo-α-L-idopyranosyluronique -(1→4)-1,6-anhydro-2-déoxy-2-sulfoamino-β-D-glucopyranose, sel d'heptasodium) : Spectre proton dans D₂O, 400MHz, T=298K, δ en ppm: 3,25 (1H, m, H2"), 3,42 (1H, dd, J=4 et 1Hz, H2), 3,60 (1H, m, H3"), entre 3,70 et 4,70 (14H, massif, H3/H4/H6, H2'/H3'/H4'/H5', H4"/H5"/H6", H2"'/H3"'), 4,75 (1H, m, H5), entre 5,20 et 5,40 (2H, m, H1' et H1"), 5,45 (1H, m, H1"'), 5,52 (1H, m, H1), 5,94 (1H, d, J=5Hz, H4)].

### EXEMPLE 3

Dans un réacteur maintenu à 62°C, on introduit 16,7 ml d'une solution d'hydroxyde de sodium à 0,0063 mol/l. Le pH de la solution est alors mesuré et pris comme valeur cible (pH = 11,7). Sous agitation, on ajoute en une fois 100 mg de l'oligosaccharide de formule (II) pour lequel n est égal à 2, R₁, R₂, R₃, R₅ et R₆ représentent un radical SO₃Na, R₄ représente un atome d'hydrogène et M est sodium. Le pH est alors régulé et maintenu à pH 11,7 par addition continue d'une solution d'hydroxyde de sodium à 0,5 mol/l. Après 10 heures on arrête l'ajout d'hydroxyde de sodium et le mélange réactionnel est refroidi à 25°C. Le pH de la solution est alors ramené entre 6 et 7 par addition de résine Amberlite IR120. Le mélange est filtré sur membrane Whatman GF/B puis concentré à sec sous pression réduite (2,7 kPa), à une température voisine de 25°C. Le produit est repris avec 3 ml d'eau distillée puis lyophilisé. On obtient ainsi 108 mg de l'oligosaccharide de formule (I) pour lequel n est égal à 2, R₁, R₂, R₃, R₅, R₆ représentent un radical SO₃Na, R₄ représente un atome d'hydrogène et M est sodium, sous forme d'un mélange des diastéréoisomères. On note de A à F les sucres constitutifs des hexasaccharides, A étant le résidu 1,6-anhydro et F le résidu acide uronique insaturé. [(acide 4-déoxy-2-*O*-sulfo-α-L-thréo-hex-4-enopyranosyluronique -(1→4)- 2-déoxy-2-sulfoamino-6-*O*-sulfo-α-D-glucopyranosyl-(1→4)- acide 2-*O-*sulfo-α-L-idopyranosyluronique -(1→4)- 2-déoxy-2-sulfoamino-6-*O*-sulfo-α-D-glucopyranosyl-(1→4)-acide 2-*O*-sulfo-α-L-idopyranosyluronique-(1→4)-1,6-anhydro-2-déoxy-2-sulfoamino-β-D-mannopyranose, sel d'undécasodium) : Spectre proton dans D₂*O*, 600MHz, T=298K, δ en ppm: 3,15 (1H, s, H2(A)), 3,25 (2H, m, H2(C+E)), 3,60 (2H, m, H3(C+E)), entre 3,65 et 4,50 (19H, massif, H2(B+D)/H3(A+B+D+F)/H4(A+B+C+D+E)/HS(C+E)/H6(A+C+E)), 4,60 (1H, s, H2(F)), 4,80 (3H, m, H5(A+B+D), 5,18 (1H, s, H1(D)), 5,30 (1H, s, H1(B)), 5,34 (1H, d, H1(C)), 5,36 (1H, d, H1(E)), 5,46 (1H, s, H1(F)), 5,57 (1H, s, H1(A)), 5,95 (1H, d, J=5Hz, H4(F)) ; (acide 4-déoxy-2-*O*-sulfo-α-L-thréo-hex-4-enopyranosyluronique-(1→4)-2-déoxy-2-sulfoamino-6-*O*-sulfo-α-D-glucopyranosyl-(1→4)-acide 2-*O*-sulfo-α-L-idopyranosyluronique -(1→4)-2-déoxy-2-sulfoamino-6-*O*-sulfo-α-D-glucopyranosyl-(1→4)-acide 2-*O*-sulfo-α-L-idopyranosyluronique-(1→4)-1,6-anhydro-2-déoxy-2-sulfoamino-β-D-glucopyranose, sel d'undécasodium) : Spectre proton dans D₂O, 600MHz, T=298K, δ en ppm: 3,25 (2H, m, H2(C+E)), 3,42 (1H, m, H2(A)), 3,60 (2H, m, H3(C+E)), entre 3,65 et 4,50 (19H, massif, H2(B+D)/H3(A+B+D+F)/H4(A+B+C+D+E)/H5(C+E)/H6(A+C+E)), 4,60 (1H, s, H2(F)), 4,80 (3H, m, H5(A+B+D), 5,18 (1H, s, H1(D)), 5,31 (1H, s, H1(B)), 5,34 (1H, d, H1(C)), 5,36 (1H, d, H1(E)), 5,46 (1H, s, H1(F)), 5,52 (1H, s, H1(A)), 5,95 (1H, d, J=5Hz, H4(F))].

### EXEMPLE 4

Dans un réacteur maintenu à 62°C, on introduit 4 ml d'une solution d'hydroxyde de sodium à 0,0316 mol/l. Le pH de la solution est alors mesuré et pris comme valeur cible (pH = 11,8). Sous agitation, on ajoute en une fois 100,8 mg un mélange d'oligosaccharides de formule (II) pour lequel n est égal à 2 comprenant 55 % de dérivé ΔIs-Is-Is (R₁, R₂, R₃, R₅ et R₆ représentent un radical SO₃Na, R₄ représente un atome d'hydrogène et M est sodium), 35 % de ΔIs-Is-IIs (R₁, R₂, R₃, et R₆ représentent un radical SO₃Na, R₅ représente soit un radical SO₃Na ou un atome d'hydrogène, R₄ représente un atome d'hydrogène et M est sodium) et 10 % de ΔIs-Is-IIIs (R₁, R₂, R₃, R₅ et R₆ représentent un radical SO₃Na, R₄ représente un atome d'hydrogène et M est sodium, la fonction SO₃M du carbone C6 étant remplacée par un hydrogène). Le pH est alors régulé et maintenu à pH 11,8 par addition continue d'une solution d'hydroxyde de sodium à 0,5 mol/l. Après 11 heures, on arrête l'ajout d'hydroxyde de sodium et le mélange réactionnel est refroidi à 25°C. Le pH de la solution est alors ramené entre 6 et 7 par addition de résine Amberlite IR120. Le mélange est filtré sur membrane Whatman GF/B puis concentré à sec sous pression réduite (2,7 kPa), à une température voisine de 25°C. Le produit est repris avec 1,5 ml d'eau distillée puis lyophilisé. On obtient ainsi 110 mg d'un mélange d'oligosaccharides de formule (I) pour lequel n est égal à 2, contenant notamment le dérivé 1,6 anhydro ΔIs-Is-Is (R₁, R₂, R₃, R₅, R₆ représentent un radical SO₃Na, R₄ représente un atome d'hydrogène et M est sodium) et le dérivé 1,6 anhydro ΔIs-Is-IIs (R₁, R₂, R₃, R₆, représentent un radical SO₃Na, R₅ représente soit un radical SO₃Na ou un atome d'hydrogène, R₄ représente un atome d'hydrogène et M est sodium). L'analyse CLHP (Chromatographie Liquide Haute Performance) en mode paire d'ions permet de suivre la transformation en dérivés de formule (I). Dans ce cas, le dosage CLHP montre que la transformation est réalisée pour les dérivés ΔIs-Is-Is, ΔIs-Is-IIs.

### EXEMPLE 5

Dans un réacteur maintenu à 66°C, on introduit 8,6 ml d'une solution d'hydroxyde de lithium à 0,025 mol/l. Le pH de la solution est alors mesuré et pris comme valeur cible (pH = 11,68). Sous agitation, on ajoute en une fois 50 mg de l'oligosaccharide de formule (II) pour lequel n est égal à 0, R₁ et R₆ représentent un radical SO₃Na et M est sodium. Le pH est alors régulé et maintenu à pH 11,68 par addition continue d'une solution d'hydroxyde de lithium à 0,466 mol/l. Après 8 heures, on arrête l'ajout d'hydroxyde de lithium et le mélange réactionnel est refroidi à 25°C. L'analyse CLHP (Chromatographie Liquide Haute Performance) en mode paire d'ions permet de suivre la transformation en dérivé de formule (I) pour lequel n est égal à 0, R₁ et R₆ représentent un radical SO₃Na et M est sodium ou lithium. Dans ce cas, le dosage CLHP montre que la transformation est réalisée à 100%. Le rendement par étalonnage externe est de 81,2%.

### EXEMPLE 6

Dans un réacteur maintenu à 66°C, on introduit 8,3 ml d'une solution d'hydroxyde de potassium à 0,0063 mol/l. Le pH de la solution est alors mesuré et pris comme valeur cible (pH = 11,1). Sous agitation, on ajoute en une fois 50 mg de l'oligosaccharide de formule (II) pour lequel n est égal à 0, R₁ et R₆ représentent un radical SO₃Na et M est sodium. Le pH est alors régulé et maintenu à pH 11,1 par addition continue d'une solution d'hydroxyde de potassium à 0,515 mol/l. Après 24 heures, on arrête l'ajout d'hydroxyde de potassium et le mélange réactionnel est refroidi à 25°C. L'analyse CLHP (Chromatographie Liquide Haute Performance) en mode paire d'ions permet de suivre la transformation en dérivé de formule (I) pour lequel n est égal à 0, R₁ et R₆ représentent un radical SO₃Na et M est sodium ou potassium. Dans ce cas, le dosage CLHP montre que la transformation est réalisée à 100%. Le rendement par étalonnage externe est de 75,6 %.

### EXEMPLE 7

Dans un réacteur maintenu à 66°C, on introduit 8,3 ml d'une solution d'hydroxyde de césium à 0,0063 mol/l. Le pH de la solution est alors mesuré et pris comme valeur cible (pH = 10,75). Sous agitation, on ajoute en une fois 50 mg de l'oligosaccharide de formule (II) pour lequel n est égal à 0, R₁ et R₆ représentent un radical SO₃Na et M est sodium. Le pH est alors régulé et maintenu à pH 10,75 par addition continue d'une solution d'hydroxyde de césium à 0,476 mol/l. Après 20 heures, on arrête l'ajout d'hydroxyde de césium et le mélange réactionnel est refroidi à 25°C. L'analyse CLHP (Chromatographie Liquide Haute Performance) en mode paire d'ions permet de suivre la transformation en dérivé de formule (I) pour lequel n est égal à 0, R₁ et R₆ représentent un radical SO₃Na et M est sodium ou césium. Dans ce cas, le dosage CLHP montre que la transformation est réalisée à 90,3%. Le rendement par étalonnage externe est de 73 %.

### EXEMPLE 8

Dans un réacteur maintenu à 66°C, on introduit 8,3 ml d'une solution d'hydroxyde de tétrabutylammonium à 0,0063 mol/l. Le pH de la solution est alors mesuré et pris comme valeur cible (pH = 10,95). Sous agitation, on ajoute en une fois 50 mg de l'oligosaccharide de formule (II) pour lequel n est égal à 0, R₁ et R₆ représentent un radical SO₃Na et M est sodium. Le pH est alors régulé et maintenu à pH 10,95 par addition continue d'une solution d'hydroxyde de tétrabutylammonium à 0,521 mol/l. Après 16 heures, on arrête l'ajout d'hydroxyde de césium et le mélange réactionnel est refroidi à 25°C. L'analyse CLHP (Chromatographie Liquide Haute Performance) en mode paire d'ions permet de suivre la transformation en dérivé de formule (I) pour lequel n est égal à 0, R₁ et R₆ représentent un radical SO₃Na et M est sodium ou tétrabutylammonium. Dans ce cas, le dosage CLHP montre que la transformation est réalisée à 96,7 %. Le rendement par étalonnage externe est de 65 %.

Les médicaments selon l'invention comprennent en tant que principe actif au moins un oligosaccharide de formule (I) ou un mélange d'oligosaccharides de formule (I), sous forme d'une composition dans laquelle il est associé à tout autre produit pharmaceutiquement compatible, pouvant être inerte ou physiologiquement actif. Les médicaments selon l'invention peuvent être employés par voie intraveineuse, sous-cutanée, orale, rectale, topique ou pulmonaire (inhalation).

Les compositions stériles pour administration intraveineuse ou sous-cutanée, sont généralement des solutions aqueuses. Ces compositions peuvent également contenir des adjuvants, en particulier des agents mouillants, isotonisants, émulsifiants, dispersants et stabilisants. La stérilisation peut se faire de plusieurs façons, par exemple par filtration aseptisante, en incorporant à la composition des agents stérilisants, par irradiation. Elles peuvent également être préparées sous forme de compositions solides stériles qui peuvent être dissoutes au moment de l'emploi dans de l'eau stérile ou tout autre milieu stérile injectable.

Comme compositions solides pour administration orale, peuvent être utilisés des comprimés, des pilules, des poudres (capsules de gélatine, cachets) ou des granulés. Dans ces compositions, le principe actif est mélangé à un ou plusieurs diluants inertes, tels que amidon, cellulose, saccharose, lactose ou silice, sous courant d'argon. Ces compositions peuvent également comprendre des substances autres que les diluants, par exemple un ou plusieurs lubrifiants tels que le stéarate de magnésium ou le talc, un agent favorisant l'absorption orale, un colorant, un enrobage (dragées) ou un vernis.

Comme compositions liquides pour administration orale, on peut utiliser des solutions, des suspensions, des émulsions, des sirops et des élixirs pharmaceutiquement acceptables contenant des diluants inertes tels que l'eau, l'éthanol, le glycérol, les huiles végétales ou l'huile de paraffine. Ces compositions peuvent comprendre des substances autres que les diluants, par exemple des produits mouillants, édulcorants, épaississants, aromatisants ou stabilisants.

Les compositions pour administration rectale sont les suppositoires ou les capsules rectales qui contiennent, outre le produit actif, des excipients tels que le beurre de cacao, des glycérides semi-synthétiques ou des polyéthylèneglycols.

Les compositions pour administration topique peuvent être par exemple des crèmes, lotions, collyres, collutoires, gouttes nasales ou aérosols.

Les doses dépendent de l'effet recherché, de la durée du traitement et de la voie d'administration utilisée; elles sont généralement comprises entre 0,5 mg et 10 mg par kg par jour par voie sous-cutanée soit 3 à 60 mg par jour pour un adulte de 60 kg.

D'une façon générale, le médecin déterminera la posologie appropriée en fonction de l'âge, du poids et de tous les autres facteurs propres au sujet à traiter.

Il est décrit également une méthode de prévention ou de traitement de maladies liées à un processus inflammatoire impliquant la production de substances cytotoxiques telle que le nitrite oxyde (NO). Les oligosaccharides de formule (I) peuvent ainsi être utilisées pour la prévention et/ou le traitement des maladies neurodégénératives pour lesquelles l'inflammation cérébrale joue un rôle délétère pouvant conduire à la mort parmi lesquelles on peut citer les ischémies du système nerveux central, les ischémies cérébrales, les ischémies de la rétine et de la cochlée, les ischémies cardiaques (infarctus du myocarde), les ischémies périphériques, les traumatismes du système nerveux central et notamment les traumatismes crâniens, spinaux et cranio-spinaux, les traumatismes de la rétine et de la cochlée, la sclérose en plaques, les douleurs neuropathiques et les neuropathies périphériques, les maladies du motoneurone dont la sclérose latérale amyotrophique, le neuro-sida, la maladie d'Alzheimer, la maladie de Parkinson et la Chorée de Huntington et certaines formes d'ostéoarthrites notamment à localisation articulaire.

## Revendications

1. Oligosaccharides de formule : 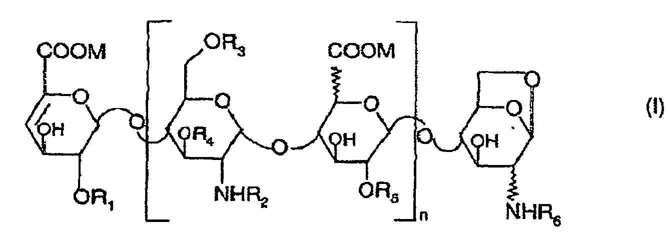 dans laquelle n est un entier de 0 à 25, R₁, R₃, R₄ et R₅, identiques ou différents, représentent un atome d'hydrogène ou un radical SO₃M, R₂ et R₆, identiques ou différents, représentent un atome d'hydrogène ou un radical SO₃M ou COCH₃ et M est sodium, calcium, magnésium ou potassium.

2. Oligosaccharides de formule (I) selon la revendication 1 pour lesquels R₄ représente un atome d'hydrogène.

3. Oligosaccharides de formule (I) selon l'une des revendications 1 ou 2 pour lesquels n est 0 ou un entier de 1 à 10.

4. Oligosaccharides de formule (I) selon l'une des revendications 1 ou 2 pour lesquels n est 0 ou un entier de 1 à 6.

5. Oligosaccharides de formule (I) selon l'une des revendications 1 ou 2 pour lesquels n est un entier de 1 à 6.

6. Oligosaccharides de formule (I) selon la revendication 1 dans laquelle n est égal à 0.

7. Oligosaccharides de formule (I) selon la revendication 1 dans laquelle n est égal à 1.

8. Oligosaccharides de formule (I) selon la revendication 1 dans laquelle :
- n est égal à 0, R₁ et R₆ représentent un radical SO₃Na et M est sodium et le mélange de ses diastéréoisomères ;
- n est égal à 1, R₁, R₂, R₃, R₅, R₆, représentent un radical SO₃Na, R₄ représente un atome d'hydrogène et M est sodium et le mélange de ses diastéréoisomères ;
- n est égal à 2, R₁, R₂, R₃, R₅, R₆ représentent un radical SO₃Na, R₄ représente un atome d'hydrogène et M est sodium et le mélange de ses diastéréoisomères ;
- n est égal à 2, R₁, R₂, R₃, R₆, représentent un radical SO₃Na, R₅ représente un atome d'hydrogène ou un radical SO₃Na, R₄ représente un atome d'hydrogène et M est sodium et le mélange de ses diastéréoisomères (dérive 1,6 anhydro ΔIs-Is-IIs).

9. Oligosaccharides de formule (I) selon l'une quelconque des revendications 1 à 8, éventuellement purifiés par chromatographie par perméation de gel de type polyacrylamide-agarose.

10. Une composition pharmaceutique comprenant un oligosacharide de formule : 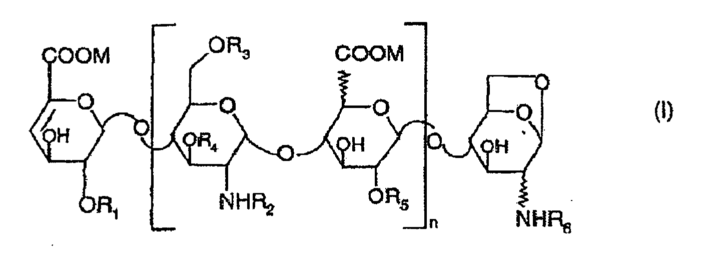 ou ses diastéréoisomères dans laquelle n est 0 ou un entier de 1 à 25, R₁, R₃, R₄ et R₅, identiques ou différents, représentent un atome d'hydrogène ou un radical SO₃M, R₂ et R₆, identiques ou différents, représentent un atome d'hydrogène ou un radical SO₃M ou COCH₃ et M est sodium, calcium, magnésium ou potassium,

11. Une composition pharmaceutique comprenant un oligosaccharide de formule (I) ou ses diastéréoisomères selon la revendication 10 dans laquelle R₄ représente un atome d'hydrogène.

12. Une composition pharmaceutique comprenant un oligosaccharide de formule (I) ou ses diastéréoisomères selon la revendication 10 dans laquelle n est 0 ou un entier de 1 à 10.

13. Une composition pharmaceutique comprenant un oligosaccharide de formule (I) ou ses diastéréoisomères selon la revendication 10 dans laquelle n est égal à 0 ou un entier de 1 à 6.

14. Une composition pharmaceutique comprenant un oligosaccharide de formule (I) ou ses diastéréoisomères selon la revendication 10 dans laquelle n est un entier de 1 à 6.

15. Une composition pharmaceutique comprenant un oligosaccharide de formule (I) ou ses diastéréoisomères selon la revendication 10 dans laquelle n est égal à 0.

16. Une composition pharmaceutique comprenant un oligosaccharide de formule (I) ou ses diastéréoisomères selon la revendication 10 dans laquelle n est égal à 1.

17. Procédé de préparation des oligosaccharides de formule (I) 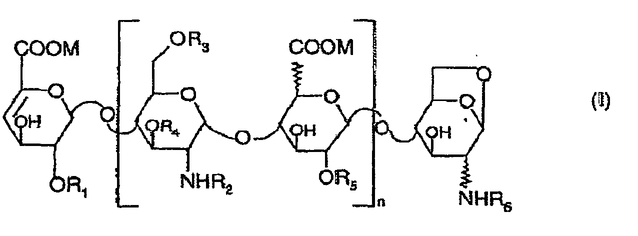 ou leurs mélanges dans laquelle n est 0 ou un entier de 1 à 25, R₁, R₃, R₄ et R₅, identiques ou différents, représentent un atome d'hydrogène ou un radical SO₃M, R₂ et R₆, identiques ou différents, représentent un atome d'hydrogène ou un radical SO₃M ou COCH₃ et M est sodium, calcium, magnésium ou potassium, **caractérisé en ce que** l'on fait réagir un hydroxyde de métal alcalin ou d'ammonium quaternaire sur des oligosaccharides de formule : 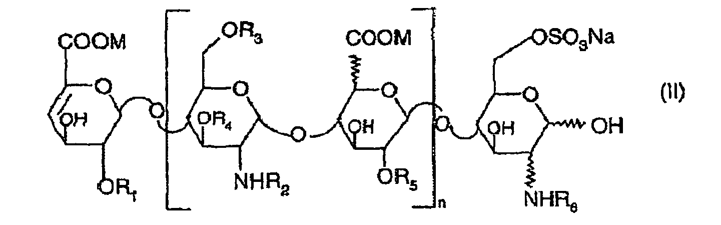 ou un mélange de ceux-ci dans laquelle n, R₁, R₂, R₃, R₄, R₅, R₆ et M, sont tels que définis plus haut.

18. Procédé selon la revendication 17 **caractérisé en ce que** l'un effectue la réaction en milieu aqueux, à une température de 40 à 80°C et à un pH de 10 à 13.

19. Procédé selon la revendication 17 **caractérisé en ce que** l'on utilise une solution aqueuse de 1 à 5% d'hydroxyde de métal alcalin ou d'ammonium quaternaire.

20. Procédé selon la revendication 17 **caractérisé en ce que** la réaction s'effectue à une température de 60 à 70°C.

21. Procédé selon la revendication 17 **caractérisé en ce que** le pH réactionnel est de 11 à 12,5.

22. Procédé selon la revendication 17 **caractérisé en ce que** l'hydroxyde de métal alcalin ou d'ammonium quaternaire est la soude, la potasse, l'hydroxyde de lithium, de césium ou l'hydroxyde de tétrabutylammonium.

23. Procédé selon la revendication 17 dans lequel les oligosaccharides de formule (I) selon la revendication 1 ou leur mélanges sont isolés.

24. Oligosaccharides de formule (I) selon l'une des revendications 1 à 9 pour utilisation pour la prévention ou le traitement de maladies liées à un processus inflammatoire impliquant la production de nitrite oxyde (NO), choisies parmi les ischémies cérébrales, cardiaques ou vasculaires périphériques, les ostéoarthrites, les traumatismes du système nerveux central, les traumatismes crâniens, spinaux et crânio-spinaux, la sclérose en plaques, les douleurs neuropathiques et les neuropathies périphériques, les maladies du motoneurone, la sclérose latérale amyotrophique, le neurosida, la maladie d'Alzheimer, la maladie de Parkinson et la Chorée de Huntington.

25. Compositions selon la revendication 10 pour utilisation pour la prévention ou le traitement de maladies liées à un processus inflammatoire impliquant la production de nitrite oxyde (NO), choisies parmi les ischémies cérébrales, cardiaques ou vasculaires périphériques, les ostéoarthrites, les traumatismes du système nerveux central, les traumatismes crâniens, spinaux et crânio-spinaux, la sclérose en plaques, les douleurs neuropathiques et les neuropathies périphériques, les maladies du motoneurone, la sclérose latérale amyotrophique, le neuro-sida, la maladie d'Alzheimer, la maladie de Parkinson et la Chorée de Huntington.

## Claims

1. Oligosaccharides of formula: 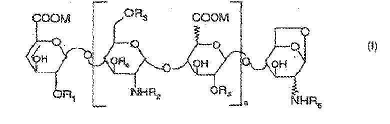 in which n is an integer from 0 to 25, R₁, R₃, R₄ and R₅, which may be identical or different, represent a hydrogen atom or a radical SO₃M, R₂ and R₆, which may be identical or different, represent a hydrogen atom or a radical SO₃M or COCH₃ and M is sodium, calcium, magnesium or potassium.

2. Oligosaccharides of formula (I) according to Claim 1, for which R₄ represents a hydrogen atom.

3. Oligosaccharides of formula (I) according to either of Claims 1 and 2, for which n is 0 or an integer from 1 to 10.

4. Oligosaccharides of formula (I) according to either of Claims 1 and 2, for which n is 0 or an integer from 1 to 6.

5. Oligosaccharides of formula (I) according to either of Claims 1 and 2, for which n is an integer from 1 to 6.

6. Oligosaccharides of formula (I) according to Claim 1, in which n is equal to 0.

7. Oligosaccharides of formula (I) according to Claim 1, in which n is equal to 1.

8. Oligosaccharides of formula (I) according to Claim 1, in which:
- n is equal to 0, R₁ and R₆ represent a radical SO₃Na and M is sodium, and the mixture of its diastereoisomers;
- n is equal to 1, R₁, R₂, R₃, R₅ and R₆ represent a radical SO₃Na, R₄ represents a hydrogen atom and M is sodium, and the mixture of its diastereoisomers;
- n is equal to 2, R₁, R₂, R₃, R₅ and R₆ represent a radical SO₃Na, R₄ represents a hydrogen atom and M is sodium, and the mixture of its diastereoisomers;
- n is equal to 2, R₁ R₂ R₃ and R₆ represent a radical SO₃Na, R₅ represents a hydrogen atom or a radical SO₃Na, R₄ represents a hydrogen atom and M is sodium, and the mixture of its diastereoisomers (1,6-anhydro ΔIs-Is-IIs derivative).

9. Oligosaccharides of formula (I) according to any one of Claims 1 to 8, optionally purified by polyacrylamide-agarose type gel permeation chromatography.

10. Pharmaceutical composition comprising an oligosaccharide of formule: 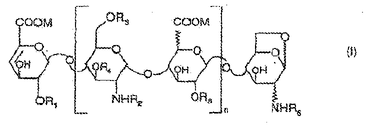 or diastereoisomers thereof, in which n is 0 or an integer from 1 to 25, R₁, R₃, R₄ and R₅, which may be identical or different, represent a hydrogen atom or a radical SO₃M, R₂ and R₆, which may be identical or different, represent a hydrogen atom or a radical SO₃M or COCH₃ and M is sodium, calcium, magnesium or potassium.

11. Pharmaceutical composition comprising an oligosaccharide of formula (I) or diastereoisomers thereof according to Claim 10, in which R₄ represents a hydrogen atom.

12. Pharmaceutical composition comprising an oligosaccharide of formula (I) or diastereoisomers thereof according to Claim 10, in which n is 0 or an integer from 1 to 10.

13. Pharmaceutical composition comprising an oligosaccharide of formula (I) or diastereoisomers thereof according to Claim 10, in which n is 0 or an integer from 1 to 6.

14. Pharmaceutical composition comprising an oligosaccharide of formula (I) or diastereoisomers thereof according to Claim 10, in which n is an integer from 1 to 6.

15. Pharmaceutical composition comprising an oligosaccharide of formula (I) or diastereoisomers thereof according to Claim 10, in which n is equal to 0.

16. Pharmaceutical composition comprising an oligosaccharide of formula (I) or diastereoisomers thereof according to Claim 10, in which n is equal to 1.

17. Process for prepaying oligosaccharides of formula (I) 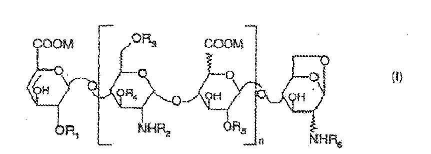 or mixtures thereof, in which n is 0 or an integer from 1 to 25, R₁, R₃, R₄ and R₅, which may be identical or different, represent a hydrogen atom or a radical SO₃M, R₂ and R₆, which may be identical or different, represent a hydrogen atom or a radical SO₃M or COCH₃ and M is sodium, calcium, magnesium or potassium, **characterized in that** an alkali metal hydroxide or quaternary ammonium hydroxide is reacted with oligosaccharides of formula: 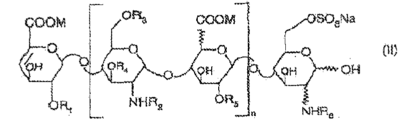 or a mixture thereof, in which n, R₁, R₂, R₃, R₄, R₅, R₆ and M are as defined above.

18. Process according to Claim 17, **characterized in that** the reaction is performed in aqueous medium, at a temperature from 40 to 80°C and at a pH of 10 to 13.

19. Process according to Claim 17, **characterized in that** an aqueous solution containing from 1% to 5% of alkali metal hydroxide or quaternary ammonium hydroxide is used.

20. Process according to Claim 17, **characterized in that** the reaction is performed at a temperature from 60 to 70°C.

21. Process according to Claim 17, **characterized in that** the reaction pH is from 11 to 12.5.

22. Process according to Claim 17, **characterized in that** the alkali metal hydroxide or quaternary ammonium hydroxide is sodium hydroxide, potassium hydroxide, lithium hydroxide, caesium hydroxide or tetrabutylammonium hydroxide.

23. Process according to Claim 17, in which the oligosaccharides of formula (I) according to Claim 1 or mixtures thereof are isolated.

24. Oligosaccharides of formula (I) according to one of Claims 1 to 9, used for preventing or treating diseases associated with an inflammatory process involving the production of nitric oxide (NO), chosen from cerebral, cardiac or peripheral vascular ischaemia, osteoarthritis, central nervous system trauma, cranial, spinal and craniospinal trauma, multiple sclerosis, neuropathic pain and peripheral neuropathies, motor neutron diseases, amyotrophic lateral sclerosis, neuro-AIDS, Alzheimer's disease, Parkinson's disease and Huntington's chorea.

25. Compositions according to Claim 10, used for preventing or treating diseases associated with an inflammatory process involving the production of nitric oxide (NO), chosen from cerebral, cardiac or peripheral vascular ischaemia, osteoarthritis, central nervous system trauma, cranial, spinal and craniospinal trauma, multiple sclerosis, neuropathic pain and peripheral neuropathies, motor neuron diseases, amyotrophic lateral sclerosis, neuro-AIDS, Alzheimer's disease, Parkinson's disease and Huntington's chorea.

## Patentansprüche

1. Oligosaccharide der Formel: 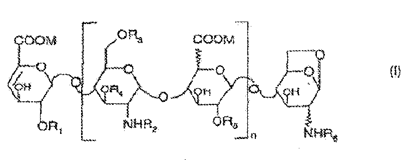 worin n eine ganze Zahl von 0 bis 25 ist, R₁, R₃, R₄ und R₅, die gleich oder verschieden sind, für ein Wasserstoffatom oder einen Rest SO₃M stehen, R₂ und R₆, die gleich oder verschieden sind, für ein Wasserstoffatom oder einen Rest SO₃M oder COCH₃ stehen und M für Natrium, Calcium, Magnesium oder Kalium steht.

2. Oligosaccharide der Formel (I) nach Anspruch 1, worin R₄ für ein Wasserstoffatom steht.

3. Oligosaccharide der Formel (I) nach einem der Ansprüche 1 oder 2, worin n für 0 oder eine ganze Zahl von 1 bis 10 steht.

4. Oligosaccharide der Formel (I) nach einem der Ansprüche 1 oder 2, worin n für 0 oder eine ganze Zahl von 1 bis 6 steht.

5. Oligosaccharide der Formel (I) nach einem der Ansprüche 1 oder 2, worin n eine ganze Zahl von 1 bis 6 ist.

6. Oligosaccharide der Formel (I) nach Anspruch 1, worin n gleich 0 ist.

7. Oligosaccharide der Formel (I) nach Anspruch 1, worin n gleich 1 ist.

8. Oligosaccharide der Formel (I) nach Anspruch 1, worin:
n gleich 0 ist, R₁ und R₆ für einen Rest SO₃Na stehen und M für Natrium steht, und das Gemisch ihrer Diastereoisomere;
- n gleich 1 ist, R₁, R₂, R₃, R₅, R₆ für einen Rest SO₃Na stehen, R₄ für ein Wasserstoffatom steht und M für Natrium steht, und das Gemisch ihrer Diastereoisomere;
- n gleich 2 ist, R₁, R₂, R₃, R₅, R₆ für einen Rest SO₃Na stehen, R₄ für ein Wasserstoffatom steht und M für Natrium steht, und das Gemisch ihrer Diastereoisomere;
- n gleich 2 ist, R₁, R₂, R₃, R₆ für einen Rest SO₃Na stehen, R₅ für ein Wasserstoffatom oder einen Rest SO₃Na steht, R₄ für ein Wasserstoffatom steht und M für Natrium steht, und das Gemisch ihrer Diastereoisomere (1,6-Anhydro-ΔIs-Is-IIS-Derivat).

9. Oligosaccharide der Formel (I) nach einem der Ansprüche 1 bis 8, die gegebenenfalls mittels Gelpermeationschromatographie des Polyacrylamid-Agarose-Typs gereinigt werden.

10. Pharmazeutische Zusammensetzung, die ein Oligosaccharid der Formel: 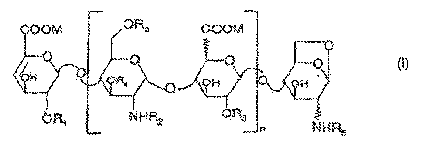 oder seine Diastereoisomere umfasst, worin n für 0 steht oder eine ganze Zahl von 1 bis 25 ist, R₁, R₃, R₄ und R₃, die gleich oder verschieden sind, für ein Wasserstoffatom oder einen Rest SO₃M stehen, R₂ und R₆, die gleich oder verschieden sind, für ein Wasserstoffatom oder einen Rest SO₃M oder COCH₃ stehen und M für Natrium, Calcium, Magnesium oder Kalium steht.

11. Pharmazeutische Zusammensetzung, die ein oligosaccharid der Formel (I) oder seine Diastereoisomere nach Anspruch 10 umfasst, worin R₄ für ein Wasserstoffatom steht.

12. Pharmazeutische Zusammensetzung, die ein Oligosaccharid der Formel (I) oder seine Diastereoisomere nach Anspruch 10 umfasst, worin n für 0 oder eine ganze Zahl von 1 bis 10 steht.

13. Pharmazeutische Zusammensetzung, die ein Oligosaccharid der Formel (I) oder seine Diastereoisomere nach Anspruch 10 umfasst, worin n für 0 oder eine ganze Zahl von 1 bis 6 steht.

14. Pharmazeutische Zusammensetzung, die ein Oligosaccharid der Formel (I) oder seine Diastereoisomere nach Anspruch 10 umfasst, worin n eine ganze Zahl von 1 bis 6 ist.

15. Pharmazeutische Zusammensetzung, die ein Oligosaccharid der Formel (I) oder seine Diastereoisomere nach Anspruch 10 umfasst, worin n gleich 0 ist.

16. Pharmazeutische Zusammensetzung, die ein Oligosaccharid der Formel (I) oder seine Diastereoisomere nach Anspruch 10 umfasst, worin n gleich 1 ist.

17. Verfahren zur Herstellung von Oligosacchariden der Formel (I) 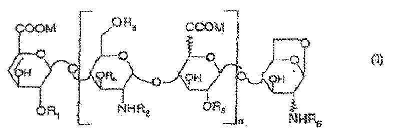 oder ihrer Gemische, worin n für 0 steht oder eine ganze Zahl von 1 bis 25 ist, R₁, R₃, R₄ und R₅, die gleich oder verschieden sind, für ein Wasserstoffatom oder einen Rest SO₃M stehen, R₂ und R₆, die gleich oder verschieden sind, für ein Wasserstoffatom oder einen Rest SO₃M oder COCH₃ stehen und M für Natrium, Calcium, Magnesium oder Kalium steht, **dadurch gekennzeichnet, dass** man ein Alkalimetallhydroxid oder quaternäres Ammoniumhydroxid mit Oligosacchariden der Formel : 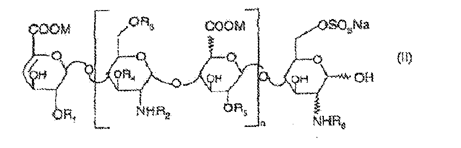 oder einem Gemisch davon, worin n, R₁, R₂, R₃, R₄, R₅, R₆ und M wie vorstehend definiert sind, umsetzt.

18. Verfahren nach Anspruch 17, **dadurch gekennzeichnet, dass** man die Umsetzung in wässrigem Medium bei einer Temperatur von 40 bis 80°C und bei einem pH von 10 bis 13 durchführt.

19. Verfahren nach Anspruch 17, **dadurch gekennzeichnet, dass** man eine wässrige Lösung von 1 bis 5% Alkalimetallhydroxid oder quaternärem Ammoniumhydroxid verwendet.

20. Verfahren nach Anspruch 17, **dadurch gekennzeichnet, dass** man die Umsetzung bei einer Temperatur von 60 bis 70°C durchführt.

21. Verfahren nach Anspruch 17, **dadurch gekennzeichnet, dass** der Reaktions-pH von 11 bis 12,5 beträgt.

22. Verfahren nach Anspruch 17, **dadurch gekennzeichnet, dass** es sich bei dem Alkalimetallhydroxid oder quaternären Ammoniumhydroxid um Natronlauge, Kalilauge, Lithium-, Cäsiumhydroxid oder Tetrabutylammoniumhydroxid handelt.

23. Verfahren nach Anspruch 17, wobei die Oligosaccharide der Formel (I) nach Anspruch 1 oder ihre Gemische isoliert werden.

24. Oligosaccharide der Formel (I) nach einem der Ansprüche 1 bis 9 für die Verwendung zur Prävention oder Behandlung von Erkrankungen, die mit einem Entzündungsvorgang zusammenhängen, an dem die Produktion von Stickoxid (NO) beteiligt ist, ausgewählt aus Ischämien des Gehirns, des Herzens und der peripheren Gefäße, Osteoarthriden, Traumatismen des zentralen Nervensystems, cranialen, spiralen und cranio-spinalen Traumatismen, multipler Sklerose, neuropathischen Schmerzen und peripheren Neuropathien, Motoneuronerkrankungen, amyotropher Lateralsklerose, Neuro-Aids, Alzheimer-Krankheit, Parkinson-Krankheit und Chorea Huntington.

25. Zusammensetzungen nach Anspruch 10 für die Verwendung zur Prävention oder Behandlung von Erkrankungen, die mit einem Entzündungsvorgang zusammenhängen, an dem die Produktion von Stickoxid (NO) beteiligt ist, ausgewählt aus Ischämien des Gehirns, des Herzens und der peripheren Gefäße, Osteoarthriden, Traumatismen des zentralen Nervensystems, cranialen, spinalen und cranio-spinalen Traumatismen, multipler Sklerose, neuropathischen Schmerzen und peripheren Neuropathien, Motoneuronerkrankungen, amyotropher Lateralsklerose, Neuro-Aids, Alzheimer-Krankheit, Parkinson-Krankheit und Chorea Huntington.
